# EUROPÄISCHE PATENTANMELDUNG

(11) **EP 2 221 043 A1**
(43) Veröffentlichungstag der Anmeldung: **25.08.2010**
(21) Anmeldenummer: 09002489.4
(22) Anmeldetag: 21.02.2009
(51) Int. Cl.: A61K 8/81, A61Q 5/06, A61K 8/87

(54) **Haarfestiger-Zusammensetzung**

(71) Anmelder: Bayer MaterialScience AG, 51368 Leverkusen (DE)
(72) Erfinder: Viala, Sophie, 50935 Köln (DE); Dörr, Sebastian, 40597 Düsseldorf (DE); Hofacker, Steffen, 51519 Odenthal (DE)

(57) **Zusammenfassung**

Die vorliegende Erfindung betrifft Haarfestiger-Zusammensetzungen, enthaltend die Kombination eines spezielle Polyurethans und eines Vinylpyrrolidon Homo- oder Copolymers sowie die Verwendung der genannten Kombination zur Herstellung von Haarfestiger-Zusammensetzungen.

## Beschreibung

Die vorliegende Erfindung betrifft Haarfestiger-Zusammensetzungen, enthaltend eine Kombination spezieller Polyurethane und Vinylpyrrolidon Homo- oder Copolymers sowie die Verwendung der genannten Kombination zur Herstellung von Haarfestiger-Zusammensetzungen.

Zur Gestaltung und Stabilisierung vielseitiger Frisuren werden Produkte eingesetzt, die als Haarfestiger (engl. Hair Styling) bekannt sind. Haarfestiger gibt es zumeist in Form von Schaumfestigern oder Haarsprays. Schaumfestiger und Haarsprays unterscheiden sich kaum in ihrer Zusammensetzung aber in ihrer Anwendung. Schaumfestiger werden im feuchten Haar als Hilfsmittel zur Modellierung der Frisur aufgetragen. Im Gegensatz dazu, sprüht man die Haarsprays auf trockene fertig gestylte Haare zur Fixierung der Frisur. Neben Haarsprays und Schaumfestiger werden auch Haarfestigergele angeboten.

In dem Falle von Haarsprays und Schaumfestigern liegen die Mittel zur Fixierung oder Gestaltung der Frisur üblicherweise als aus Aerosolbehältern, Quetschflaschen oder durch eine Pump-, Sprüh- oder Schäumvorrichtungen versprühbare Präparate vor, die aus einer alkoholischen oder wäßrigalkoholischen Lösung von filmbildenden natürlichen oder synthetischen Polymeren bestehen. Diese Polymere können aus der Gruppe der nichtionischen, kationischen, amphoteren oder anionischen Polymeren ausgewählt werden. Im Falle von Haarfestigergelen werden die oben beschriebenen Präparate mit herkömmlichen Verdickern auf eine akzeptable Viskosität eingestellt.

Seit Jahren ist der Trend zu stark gestylten Haaren. Um solche Frisuren zu gestalten, werden filmbildende Polymere eingesetzt, die ein starkes Haltevermögen aufweisen. Im Stand der Technik werden Polyvinylppyrrolidone oder Vinylpyrrolidoncopolymere insbesondere Vinylpyrrolidon/Vinylacetat-Copolymer bevorzugt verwendet. Der Einsatz von solchen filmbildenden Polymeren in Haarfestigerformulierungen führt zu Nachteilen wie unnatürlichen Frisuren (sogenannte "Betonfrisuren"), unangenehmer Haptik, Schuppenbildung nach dem Kämmen, etc, wie dem Fachmann bekannt ist.

Außerdem zeigen die herkömmlichen filmbildenden Polymeren eine geringe Feuchtigkeits- und/oder Wasserresistenz, wenn die Haare im Kontakt mit Regen oder Schweiß stehen, bzw. bei Feuchtigkeitskontakt oder unter dem Einfluss hoher Luftfeuchte z.B. während des Badens.

Die Verwendung der Kombination von Polyurethandispersionen und wasserlöslichen Polymeren ist in EP-A 950 416 beschrieben. Bevorzugt werden als wasserlösliche Polymere Polyvinylpyrrolidone oder Copolymere eingesetzt, die aus Vinylpyrrolidon- und Vinylester-Monomeren gebildet sind. Der Nachteil bei dem Einsatz eines wasserdispergierbaren Polyurethans nach der Anwendung ist die schlechte Auswaschbarkeit von den Haaren.

Es wurde nun in überraschender Weise gefunden, dass gerade eine Kombination von Vinylpyrrolidon-Copolymeren mit Polyurethanen basierend auf in Wasser nicht löslichen oder dispergierbaren Prepolymeren einen flexiblen Film auf den Haaren bildet und gleichzeitig ein ausgezeichnetes Haltvermögen aufweist und sich somit hervorragend für Haarfestigerformulierungen eignet.

Gegenstand der vorliegenden Erfindung sind somit daher kosmetische Zusammensetzungen, enthaltend eine Kombination von Vinylpyrrolidon-Copolymeren I) und Polyurethanen II), wobei letztere erhältlich sind durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B). Ebenfalls Gegenstand der vorliegenden Erfindung ist die Verwendung solcher kosmetischer Zusammensetzungen als Haarfestiger.

Haarfestiger im Sinne der Erfindung sind beispielsweise Schaumfestiger, Flüssigfestiger, Haarsprays, Stylinggele, Stylingcreme oder Schaumaerosole zur Formgebung der Haare.

Im Rahmen der Erfindung bedeutet der Begriff "wasserunlösliches, nicht wasserdispergierbares Polyurethanprepolymer" insbesondere, dass die Wasserlöslichkeit des erfindungsgemäß verwendeten Prepolymers bei 23°C weniger als 10 g / Liter, bevorzugt weniger als 5 g / Liter beträgt und das Prepolymer bei 23° in Wasser, insbesondere entionisiertem Wasser keine sedimentationsstabile Dispersion, ergibt.

Bevorzugt weist das erfindungsgemäß verwendete Polyurethanprepolymer A) endständige Isocyanatgruppen auf, d.h. die Isocyanatgruppen liegen an den Kettenenden des Prepolymers. Besonders bevorzugt weisen sämtliche Kettenenden eines Prepolymers Isocyanatgruppen auf.

Weiterhin weist das erfindungsgemäß verwendete Polyurethanprepolymer A) bevorzugt im Wesentlichen weder ionische noch ionogene Gruppen auf, d.h. der Gehalt an ionischen und ionogenen Gruppen liegt typischerweise unter 15 Milliequivalenten pro 100 g Polyurethanprepolymer A), bevorzugt unter 5 Milliequivalenten, besonders bevorzugt unter einem Milliequivalente und ganz besonders bevorzugt unter 0,1 Milliequivalenten pro 100 g Polyurethanprepolymer A).

Im Falle saurer ionischer und/oder ionogener Gruppen beträgt die Säurezahl des Prepolymers zweckmäßig unter 30 mg KOH/g Prepolymer, bevorzugt unter 10 mg KOH/g Prepolymer. Die Säurezahl gibt die Masse Kaliumhydroxid in mg an, die zur Neutralisation von 1 g der zu untersuchenden Probe erforderlich ist (Messung nach DIN EN ISO 211). Die neutralisierten Säuren, also die entsprechenden Salze weisen naturgemäß keine oder eine reduzierte Säurezahl auf. Hier ist erfindungsgemäß die Säurezahl der korrespondierenden freien Säure entscheidend.

Die aminofunktionellen Verbindungen B) werden bevorzugt aus primären und/oder sekundären Aminen und/oder Diaminen ausgewählt. Insbesondere umfassen die aminofunktionellen Verbindungen B) mindestens ein Diamin. Die aminofunktionellen Verbindungen B) werden bevorzugt aus aminofunktionellen Verbindungen B2), die ionische oder ionogene Gruppe aufweisen, und aminofunktionellen Verbindungen B1), die keine ionische oder ionogene Gruppe aufweisen, ausgewählt.

In einer besonders bevorzugten Ausführungsform der Erfindung umfassen die aminofunktionellen Verbindungen B) wenigstens eine aminofunktionelle Verbindung B2), die ionische und/oder ionogene (ionenbildende) Gruppen aufweist. Besonders bevorzugt wird als ionische und/oder ionogene Gruppe die Sulfonat- bzw. die Sulfonsäuregruppe, noch bevorzugter die Natriumsulfonatgruppe verwendet.

In einer weiteren bevorzugten Ausführungsform der Erfindung umfassen die aminofunktionellen Verbindungen B) sowohl aminofunktionelle Verbindungen B2), die ionische und/oder ionogene Gruppe aufweisen, als auch aminofunktionellen Verbindungen B1), die keine ionische oder ionogene Gruppe aufweisen.

Polyurethane im Sinne der Erfindung sind demnach polymere Verbindungen, die mindestens zwei bevorzugt mindestens drei urethangruppenhaltige Wiederholungseinheiten aufweisen:

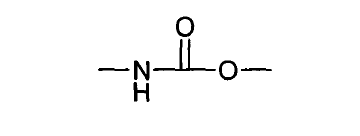

Erfindungsgemäß sind auch solche Polyurethane eingeschlossen, die herstellungsbedingt auch harnstoffgruppenhaltige Wiederholungseinheiten: 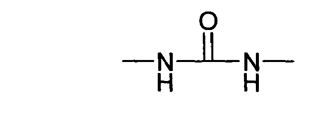 aufweisen, wie sie insbesondere bei der Umsetzung der isocyanatterminierten Prepolymere A) mit den aminofunktionellen Verbindungen B) gebildet werden.

Bei den erfindungsgemäßen kosmetischen Zusammensetzungen handelt es sich insbesondere um wasserenthaltende, d.h. wässrige Zusammensetzungen, in denen die polymeren Bestandteile I) und II) dispergiert, d.h. im Wesentlichen nicht gelöst vorliegt. Wasser bildet im Allgemeinen neben wahlweise vorhandenen anderen flüssigen Medien, wie beispielsweise Lösungsmitteln, den Hauptbestandteil (> 50 Gew.-%) der Dispergiermedien, bezogen auf die Gesamtmenge der flüssigen Dispergiermedien in den erfindungsgemäßen kosmetischen Zusammensetzungen, gegebenenfalls auch das alleinige flüssige Dispergiermedium.

Die erfindungsgemäßen kosmetischen Zusammensetzungen weisen bevorzugt einen Gehalt an flüchtigen organischen Verbindungen (VOC) von weniger als 80 Gew.-%, bevorzugter von weniger als 55 Gew.-%, noch bevorzugter von weniger als 40 Gew.-% bezogen auf die erfindungsgemäßen kosmetischen Zusammensetzungen auf.

Die zur Herstellung der erfindungsgemäßen kosmetischen Zusammensetzungen verwendeten Polyurethane in Form wässriger Dispersionen weisen bevorzugt einen Gehalt an flüchtigen organischen Verbindungen (VOC) von weniger als 10 Gew.-%, bevorzugter von weniger als 3 Gew.-%, noch bevorzugter von weniger als 1 Gew.-% bezogen auf die auf wässrige Polyurethandispersion auf.

Die Bestimmung des Gehalts an flüchtigen organischen Verbindungen (VOC) erfolgt im Rahmen der vorliegenden Erfindung insbesondere durch gaschromatographische Analyse.

Die zur Herstellung der Polyurethane verwendeten Prepolymere A) sind bevorzugt erhältlich durch die Umsetzung von einem oder mehreren Polyolen, ausgewählt aus der Gruppe, die aus Polyether-Polyolen, Polycarbonatpolyolen, Polyether-Polycarbonat-Polyolen und/oder Polyesterpolyolen besteht, und Polyisocyanaten, wie weiter unten näher erläutert wird.

Die in den erfindungsgemäßen kosmetischen Zusammensetzungen enthaltenen Polyurethane enthalten über das Prepolymer A) demnach bevorzugt mindestens eine Sequenz, die ausgewählt wird aus der Gruppe, die besteht aus: Polyether-, Polycarbonat-, Polyether-Polycarbonat- und PolyesterSequenzen. Dies bedeutet erfindungsgemäß insbesondere, dass die Polyurethane Ethergruppen- und/oder Carbonatgruppen-haltige oder Estergruppen-Wiederholungseinheiten enthalten. Die Polyurethane können beispielsweise ausschließlich Polyether-Sequenzen oder ausschließlich Polycarbonatsequenzen oder ausschließlich Polyestersequenzen enthalten. Sie können aber auch sowohl Polyether- als auch Polycarbonat-Sequenzen aufweisen, wie sie beispielsweise bei der Herstellung von Polycarbonat-Polyolen unter Verwendung von Polyetherdiolen gebildet werden, wie unten noch ausführlich beschrieben wird. Zusätzlich können sie Polyether-Polycarbonat-Sequenzen aufweisen, welche aus der Verwendung von Polyether-Polycarbonat-Polyolen, wie weiter unten näher beschrieben, hervorgehen.

Besonders bevorzugte Polyurethane werden unter Verwendung von polymeren Polyether-Polyolen und/oder polymeren Polycarbonat-Polyolen und/oder Polyether-Polycarbonat-Polyolen oder Polyesterpolyolen erhalten, welche jeweils zahlenmittlere Molekulargewichte von bevorzugt etwa 400 bis etwa 6000 g/mol (hier und bei den folgenden Molekulargewichtangaben bestimmt durch Gelpermeationschromatographie gegenüber Polystyrol-Standard in Tetrahydofuran bei 23°C) aufweisen. Ihre Verwendung bei der Herstellung der Polyurethane bzw. Polyurethanprepolymere führt durch Umsetzung mit Polyisocyanaten zur Bildung entsprechender Polyether- und/oder Polycarbonat und/oder Polyether-Polycarbonat-Sequenzen oder Polyestersequenzen in den Polyurethanen mit entsprechendem Molgewicht dieser Sequenzen. Besonders bevorzugt sind erfindungsgemäß Polyurethane, die aus polymeren Polyether-Diolen und/oder polymeren Polycarbonat-Diolen und/oder Polyether-Polycarbonat-Polyolen oder Polyesterpolyolen mit linearem Aufbau erhalten werden.

Die erfindungswesentlichen Polyurethane sind bevorzugt im Wesentlichen lineare Moleküle, können jedoch auch verzweigt sein, was weniger bevorzugt ist.

Das zahlenmittlere Molekulargewicht der erfindungsgemäß bevorzugt verwendeten Polyurethane beträgt typischerweise 1000 bis 200000 g/mol, bevorzugt 5000 bis 150000 g/mol. Molekulargewichte von oberhalb 200000 g/mol können unter Umständen nachteilig sein, da sich die erfindungsgemäßen Zusammensetzungen bei Verwendung als Haarfestiger sich manchmal schlecht auswaschen lassen.

In einer bevorzugten Ausführungsform werden die erfindungswesentlichen Polyurethane bei der Herstellung der erfindungsgemäßen kosmetischen Zusammensetzungen als wässrige Polyurethan-Dispersionen eingesetzt, die erhältlich sind, in dem
A) isocyanatfunktionelle Prepolymere aus
   A1) organischen Polyisocyanaten,
   A2) polymeren Polyolen, bevorzugt mit zahlenmittleren Molekulargewichten von 400 bis 8000 g/mol (hier und bei den folgenden Molekulargewichtangaben bestimmt durch Gelpermeationschromatographie gegenüber Polystyrol-Standard in Tetrahydofuran bei 23°C), besonders bevorzugt 400 bis 6000 g/mol und ganz besonders bevorzugt von 600 bis 3000 g/mol, und OH-Funktionalitäten von bevorzugt 1,5 bis 6, besonders bevorzugt 1,8 bis 3, ganz besonders bevorzugt von 1,9 bis 2,1,
   A3) gegebenenfalls hydroxyfunktionellen Verbindungen mit Molekulargewichten von bevorzugt 62 bis 399 g/mol, und
   A4) gegebenenfalls nichtionischen Hydrophilierungsmitteln,
      hergestellt werden, wobei die isocyanatfunktionellen Prepolymere A) einen Gehalt an ionischen und ionogenen Gruppen von weniger als15 Milliequivalenten pro 100 g Prepolymer A) aufweisen, und
B) deren freie NCO-Gruppen dann ganz oder teilweise
   mit einer oder mehreren aminofunktionellen Verbindungen B), wie primären und/oder sekundären Aminen und/oder Diaminen,
   umsetzt, wobei die so erhaltenen Polyurethane während oder nach Schritt B) in Wasser dispergiert werden.

Besondere bevorzugt erfolgt im Schritt B) die Umsetzung mit einem Diamin oder mehreren Diaminen unter Kettenverlängerung. Das isocyanatfunktionelle Prepolymer A) ist dabei vor Umsetzung mit den Komponenten B) nicht in reinem Wasser löslich oder stabil dispergierbar. Dabei können zusätzlich monofunktionelle Amine als Kettenabbrecher zur Molekulargewichtssteuerung zugesetzt werden.

Als Komponente B) können insbesondere Amine verwendet werden, die keine ionischen bzw. ionogenen, wie anionisch hydrophilierende Gruppen aufweisen (im folgenden Komponente B1)) und es werden Amine verwendet, die ionische bzw. ionogene, wie insbesondere anionisch hydrophilierenden Gruppen aufweisen (im folgenden Komponente B2)).

Bevorzugt wird im Schritt B) der Umsetzung des Prepolymers eine Mischung aus Komponente B1) und Komponente B2) zur Umsetzung gebracht. Durch die Verwendung der Komponente B1) kann eine hohe Molmasse aufgebaut werden, ohne dass die Viskosität des zuvor hergestellten isocyanatfunktionellen Präpolymers in einem Maße ansteigt, welches einer Verarbeitung entgegenstehen würde. Durch die Verwendung der Kombination der Komponenten B1) und B2) läßt sich eine optimale Balance zwischen Hydrophilie und Kettenlänge und damit eine gute Substantivität erzielen, ohne das sich "build-up"-Effekte einstellen.

Die erfindungsgemäß verwendeten Polyurethane weisen bevorzugt anionische Gruppen, bevorzugt Sulfonatgruppen auf. Diese anionischen Gruppen werden in die erfindungsgemäß verwendeten Polyurethanen über die in Schritt B) umgesetzte Aminkomponente B2) eingeführt. Die erfindungsgemäß verwendeten Polyurethane weisen gegebenenfalls zusätzlich nicht-ionische Komponenten zu Hydrophilierung auf. Besonders bevorzugt sind in den erfindungsgemäß verwendeten Polyurethanen zur Hydrophilierung ausschließlich Sulfonatgruppen enthalten, welche über entsprechende Diamine als Komponente B2) in das Polyurethan eingeführt werden.

Um eine gute Sedimentationsstabilität zu erreichen, liegt die zahlenmittlere Teilchengröße der speziellen Polyurethan-Dispersionen bevorzugt bei weniger als 750 nm, besonders bevorzugt bei weniger als 500 nm, bestimmt mittels Laserkorrelations-Spektroskopie nach Verdünnung mit entionisiertem Wasser (Gerät: Malvern Zetasizer 1000, Malver Inst. Limited).

Der Feststoffgehalt der Polyurethan-Dispersionen, welche zur Herstellung der Haarfestiger-Zusammensetzung der Erfindung bevorzugt verwendet wird, beträgt im allgemeinen 10 bis 70, bevorzugt 30 bis 65, besonders bevorzugt 40 bis 60 Gew.-%. Die Feststoffgehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

Bevorzugt weisen diese Polyurethan-Dispersionen weniger als 5 Gew.-%, besonders bevorzugt weniger als 0,2 Gew.-%, bezogen auf die Masse der Dispersionen, an ungebundenen organischen Aminen auf. Der Gehalt in den Haarfestigerzusammensetzungen ist entsprechend noch geringer.

Geeignete Polyisocyanate der Komponente A1) sind insbesondere die dem Fachmann an sich bekannten aliphatischen, aromatischen oder cycloaliphatischen Polyisocyanate einer NCO-Funktionalität von größer oder gleich 2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4-Trimethylhexamethylendiisocyanat, die isomeren Bis-(4,4'-isocyanatocyclohexyl)methane oder deren Mischungen beliebigen Isomerengehalts, 1,4-Cyclohexylendiisocyanat, 4-Isocyanatomethyl-1,8-octandüsocyanat (Nonantriisocyanat), 1,4-Phenylendiisocyanat, 2,4- und/oder 2,6-Toluylendiisoeyanat, 1,5-Naphthylendiisocyanat, 2,2'-und/oder 2,4'- und/oder 4,4'-Diphenylmethandiisocyanat, 1,3-und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysindiisocyanate) mit C1-C8-Alkylgruppen.

Neben den vorstehend genannten Polyisocyanaten können auch modifizierte Diisocyanate die eine Funktionalität ≥ 2 aufweisen, mit Uretdion-, Isocyanurat-, Urethan-, Allophanat-, Biuret-, Iminooxadiazindion- oder Oxadiazintrionstruktur sowie Mischungen aus diesen anteilig eingesetzt werden.

Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von 2 bis 4, bevorzugt 2 bis 2,6 und besonders bevorzugt 2 bis 2,4, ganz besonders bevorzugt 2.

Besonders bevorzugt werden in A1) Hexamethylendiisocyanat, Isophorondiisocyanat oder die isomeren Bis-(4,4'-isocyanatocyclohexyl)methane sowie Mischungen der vorgenannten Diisocyanate eingesetzt.

In A2) werden polymere Polyole mit einem zahlenmittleren Molekulargewicht Mₙ von bevorzugt 400 bis 8000 g/mol, bevorzugter von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Der Ausdruck "polymere" Polyole bedeutet hier insbesondere, dass die genannten Polyole mindestens zwei, bevorzugter mindestens drei miteinander verbundene Wiederholungseinheiten aufweisen.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Die bevozugt verwendeten Polyesterpolyole sind die an sich bekannten Polykondensate aus Disowie gegebenenfalls Tri- und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, Butandiol(1,3), Butandiol(1,4), Hexandiol(1,6) und Isomere, Neopentylglykol oder Hydroxypivalinsäureneopentylglykolester, wobei Hexandiol(1,6) und Isomere, Butandiol(1,4), Neopentylglykol und Hydroxypivalinsäureneopentylglykolester bevorzugt sind. Daneben können auch Polyole wie Trimethylolpropan, Glycerin, Erythrit, Pentaerythrit, Trimetylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und Phthalsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Erfindungsgemäß besonders bevorzugt sind als Komponente A2) zur Herstellung der Polyurethane, Polyesterpolyole mit einem zahlenmittleren Molekulargewicht von 600 bis 3000 g/mol, insbesondere aliphatische Polyesterpolyole auf Basis aliphatischer Carbonsäuren und aliphatischer Polyole, insbesondere auf Basis von Adipinsäure und aliphatischen Alkoholen, wie Hexandiol und/oder Neopentylglykol.

Ebenfalls können als Komponente A2) hydroxylgruppenaufweisende Polycarbonate, bevorzugt Polycarbonatdiole, mit zahlenmittleren Molekulargewichten Mₙ von bevorzugt 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält die Diolkomponente 40 bis 100 Gew.-% Hexandiol, bevorzugt sind 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden.

Hydroxylgruppen aufweisende Polycarbonate sind bevorzugt linear gebaut.

Ebenfalls können als Komponente A2) Polyetherpolyole eingesetzt werden.

Besonders geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten.

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

Besonders bevorzugte Komponenten in A2) sind Polytetramethylenglykolpolyether und Polycarbonat-Polyole bzw. deren Mischungen und besonders bevorzugt sind Polytetramethylenglykolpolyether.

In bevorzugten Ausführungsformen der Erfindung handelt es sich bei Komponente A2) demnach um:
- Mischungen, enthaltend wenigstens ein Polyether-Polyol und wenigstens ein Polycarbonat-Polyol,
- Mischungen, enthaltend mehr als ein Polyether-Polyol, bzw. ein Gemisch mehrerer Polyether-Polyole mit unterschiedlichen Molekulargewichten, wobei es sich insbesondere um Poly(tetramethylenglykol)polyetherpolyole (wie (HO-(CH₂-CH₂-CH₂-CH₂-O)ₓ-H) handelt,
- Mischungen, enthaltend mehr als ein Polyether-Polyol, und wenigstens ein PolycarbonatPolyol, sowie
- besonders bevorzugt Polyesterpolyole mit einem zahlenmittleren Molekulargewicht von 600 bis 3000 g/mol, insbesondere aliphatische Polyesterpolyole auf Basis aliphatischer Carbonsäuren und aliphatischer Polyole, insbesondere auf Basis von Adipinsäure und aliphatischen Alkoholen, wie Hexandiol und/oder Neopentylglykol,
wobei die Komponente A) definitionsgemäß im Wesentlichen weder ionische noch ionogen Gruppen aufweist.

Als Komponente A3) können wahlweise Polyole, insbesondere nicht-polymere Polyole, des bevorzugt genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Geeignet sind auch Esterdiole des genannten. Molekulargewichtsbereichs wie α-Hydroxybutyl-εhydroxy-capronsäureester, ω-Hydroxyhexyl-y-hydroxybuttersäure-ester, Adipinsäure-(β-hydroxyethyl)ester oder Terephthalsäurebis(β-hydroxyethyl)-ester.

Ferner können als Komponente A3) auch monofunktionelle isocyanatreaktive Hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykolmonomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

In einer bevorzugten Ausführungsform der Erfindung enthält dass erfindungsgemäß verwendete Polyurethan weniger als etwa 10 Gew.-% der Komponente A3), bevorzugt weniger als 5 Gew.-% der Komponente A3), jeweils bezogen auf die Gesamtmasse des Polyurethans, noch bevorzugter wird Komponente A3) zur Herstellung des Polyurethans nicht verwendet.

Als Komponente A4) werden zur Herstellung der erfindungsgemäß verwendeten Polyurethane gegebenenfalls ein oder mehrere insbesondere isocyanatreaktive nichtionische Hydrophilierungsmittel verwendet. Die als Komponente A4) verwendeten Hydrophilierungsmittel sind insbesondere von den Komponenten A2) und A3) verschieden. Das isocyantfunktionelle Prepolymer A) wird durch den Einbau von A4) nicht wasserlöslich oder wasserdispergierbar. Dies ist erst nach Durchführung von Schritt B) der Fall. Insofern ist die der eingesetzte Anteil von A4) entsprechend limitiert.

Geeignete nichtionisch hydrophilierende Verbindungen als Komponente A4) sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.

Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.

Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Die Komponente B) wird bevorzugt aus primären oder sekundären Amin und/oder Diaminen ausgewählt. Sie umfasst insbesondere Diamine.

Als Komponente B) können insbesondere Amine verwendet werden, die keine ionischen bzw. ionogenen, wie anionisch hydrophilierenden Gruppen aufweisen (im folgenden Komponente B1)), und es können Amine verwendet werden, die ionische bzw. ionogene, wie insbesondere anionisch hydrophilierenden Gruppen aufweisen (im folgenden Komponente B2)). Bevorzugt wird im Schritt B) der Umsetzung des Prepolymers eine Mischung aus der Komponente B1) und der Komponente B2) zur Umsetzung gebracht.

Beispielsweise können als Komponente B1) organische Di- oder Polyamine wie beispielsweise 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, 4,4-Diaminodicyclohexylmethan, Hydrazinhydrat, und/oder Dimethylethylendiamin eingesetzt werden.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1- methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin.

Bevorzugt werden als Komponente B1) 1,2-Ethylendiamin, Bis(4-aminocyclohexyl)methan, 1,4-Diaminobutan, Isophorondiamin, Ethanolamin, Diethanolamin und Diethylentriamin eingesetzt.

Besonders bevorzugt umfasst die Komponente B) mindestens eine Komponente B2), die anionisch hydrophilierend wirkt. Solche Amine der Komponente B2) enthalten bevorzugt eine Sulfonsäure- oder Sulfonatgruppe, besonders bevorzugt eine Natriumsulfonatgruppe. Geeignete anionisch hydrophilierende Verbindungen als Komponente B2) sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure, 1,2-oder 1,3-Propylendiamin-β-ethylsulfonsäure oder Taurin. Weiterhin kann das Salz der Cyclohexylaminopropansulfonsäure (CAPS) aus WO-A 01/88006 als anionisches Hydrophilierungsmittel verwendet werden.

Besonders bevorzugte anionische Hydrophilierungsmittel B2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure.

Besonders bevorzugt enthaltend die erfindungsgemäß verwendeten Polyurethane mindestens eine Sulfonatgruppe.

Gegebenenfalls kann die anionische Gruppe in der Komponente B2) auch eine Carboxylat bzw. Carbonsäuregruppe sein. Die Komponente B2) wird dann bevorzugt aus Diaminocarbonsäuren ausgewählt. Diese Ausführungsform ist allerdings weniger bevorzugt, da Carbonsäure-basierende Komponenten B2) in höheren Konzentrationen eingesetzt werden müssen.

Zur Hydrophilierung können auch Mischungen aus anionischen Hydrophilierungsmitteln B2) und nichtionischen Hydrophilierungsmitteln A4) verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.-% Komponente A1),
55 bis 90 Gew.-% A2),
0,5 bis 20 Gew.-% Summe der Komponenten A3) und/oder B1)
0,1 bis 25 Gew.-% Summe der Komponenten A4) und/oder B2), wobei bezogen auf die Gesamtmengen der Komponenten A1) bis A4) und B1) bis B2) besonders bevorzugt 0,1 bis 5 Gew.-% an anionischen bzw. potentiell anionischen Hydrophilierungsmitteln B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
5 bis 35 Gew.% Komponente A1),
60 bis 90 Gew.-% A2),
0,5 bis 15 Gew.% Summe der Komponenten A3) und/oder B1)
0,1 bis 15 Gew.-% Summe der Komponenten Komponente A4) und/oder B2), wobei bezogen auf die Gesamtmengen der Komponenten A1) bis A4) und B1) bis B2) besonders bevorzugt 0,2 bis 4 Gew.-% an anionischen bzw. potentiell anionischen Hydrophilierungsmitteln B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% aufaddieren:
10 bis 30 Gew.-% Komponente A1),
65 bis 85 Gew.-% A2),
0,5 bis 14 Gew.-% Summe der Komponenten A3) und/oder B1)
0,1 bis 13,5 Gew.-% Summe der Komponenten A4) und/oder B2), wobei bezogen auf die Gesamtmengen der Komponenten A1) bis A4) und B1) bis B2) besonders bevorzugt 0,5 bis 3,0 Gew.-% an anionischen bzw. potentiell anionischen Hydrophilierungsmitteln aus B2) verwendet werden.

Die Herstellung der Polyurethan-Dispersionen kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt bevorzugt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z. B. Prepolymer-Mischverfahren, Acetonverfahren oder Schmelzdispergierverfahren verwendet werden. Bevorzugt wird nach das Aceton-Verfahren angewandt.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon, besonders bevorzugt ist Aceton. Auch die Zugabe anderer Lösemittel ohne isocyanatreaktive Gruppen ist möglich, jedoch nicht bevorzugt.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanat-Gruppen zu isocyanatreaktiven Gruppen im allgemeinen 1,05 bis 3,5, bevorzugt 1,1 bis 3,0, besonders bevorzugt 1,1 bis 2,5.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder ganz besonders bevorzugt Alkalimetallbasen wie die entsprechenden Hydroxide eingesetzt.

Die Verwendung von organischen Aminen ist nicht bevorzugt.

Als Neutralisationsmittel werden bevorzugt anorganische Basen, wie wässrige Ammoniaklösung oder Natrium- bzw. Kaliumhydroxid einsetzbar.

Bevorzugt sind Natriumhydroxid und Kaliumhydroxid,

Die Stoffmenge der Basen beträgt 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, in dem das Dispergierwasser bereits das Neutralisationsmittel enthält.

Im Anschluss wird in einem weiteren Verfahrensschritt, falls noch nicht oder nur teilweise geschehen, das erhaltene Prepolymer mit Hilfe von aliphatischen Ketonen wie Aceton oder 2-Butanon gelöst.

Die Umsetzung der Komponenten A1) bis A4) zum Prepolymer erfolgt teilweise oder vollständig, bevorzugt aber vollständig. Es werden so Polyurethan-Prepolymere, die freie Isocyanatgruppen enthalten, in Substanz oder in Lösung erhalten.

Bei der Kettenverlängerung in Stufe B) werden NH₂- und/oder NH-funktionelle Komponenten mit den noch verbliebenen Isocyanatgruppen des Prepolymers umgesetzt. Bevorzugt wird die Kettenverlängerung/-terminierung vor der Dispergierung in Wasser durchgeführt.

Geeignete Komponenten B) zur Kettenverlängerung sind insbesondere organische Di- oder Polyamine B1) wie beispielsweise Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Diaminodicyclohexylmethan und/oder Dimethylethylendiamin.

Darüber hinaus können auch Verbindungen B1), die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-methylaminobutan, Alkanolamine wie N-Aminoethylethanolamin, Ethanolamin, 3-Aminopropanol, Neopentanolamin zur Kettenverlängerung bzw. -terminierung eingesetzt werden.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketim von diprimären Aminen, primär/tertiäre Amine, wie N,N-Dimethylaminopropylamin verwendet.

Werden zur Kettenverlängerung anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Der Kettenverlängerungsgrad, also das Äquivalentverhältnis von NCO-reaktiven Gruppen der zur Kettenverlängerung und Kettenterminierung eingesetzten Verbindungen zu freien NCO-Gruppen des Prepolymers, liegt im allgemeinen zwischen 40 und 150 %, bevorzugt zwischen 50 und 110%, besonders bevorzugt zwischen 60 und 100 %.

Die aminischen Komponenten B1) und B2), können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 40 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den so hergestellten Polyurethan-Dispersionen beträgt typischerweise weniger als 10 Gew.-%, bevorzugt weniger als 3 Gew.-%, bezogen auf die gesamte Dispersion.

Der pH-Wert der erfindungsgemäß verwendeten wässrigen Polyurethan-Dispersionen beträgt typischerweise weniger als 8,0, bevorzugt weniger als 7,5 und liegt besonders bevorzugt zwischen 5,5 und 7,5.

Die erfindungsgemäßen kosmetischen Zusammensetzungen enthalten neben der speziellen Polyurethanen II) Vinylpyrrolidon Homo- oder Copolymere. Hierzu gehören beispielweise Polyvinylpyrrolidon, Vinylpyrrolidon/vinylester-Copolymer wie Copolymerisate aus N-Vinylpyrrolidon und Vinylacetat und/oder Vinylpropionat in verschiedenen Konzentrationsverhältnissen, Vinylpyrrolidon/vinylacrylat-Copolymere wie Copolymere aus Vinylpyrrolidon und Dimethylaminoethylmethacrylat, Terpolymere aus Vinylcaprolactam, Vinylpyrrolidon und Dimethylaminoethylmethacrylat.

Ganz besondere bevorzugte nichtionische Polymere sind Homopolymere des Vinylpyrrolidons z. B. Luviskol® K von der Firma BASF, Copolymerisate aus Vinylpyrrolidon und Vinylacetat z. B. Luviskol® VA Typen der Firma BASF oder PVPVA® S630L der Firma ISP, Terpolymere aus Vinylpyrrolidon, Vinylacetat und Propionat wie z. B. Luviskol® VAP von BASF.

Beispiele für typische Molmassen (Mw) derartiger Polymere liegen im Bereich von 2500 bis 2500000 g/mo,l bevorzugt 50000 bis 1000000 g/mol.

Ebenfalls geeignet sind Copolymerisate aus Vinylpyrrolidon, Acrylsäure und C1-C20 Alkyl Methacrylate z.B. Acrylidone® LM der Firma ISP

Die erfindungsgemäßen kosmetischen Zusammensetzungen enthalten bezogen auf das Gesamtgewicht der kosmetischen Zusammensetzungen bevorzugt 0,1 bis 20 Gew.-%, besonders bevorzugt 0,5 bis 10 Gew.-% des oben beschriebenen Polyurethans.

Die erfindungsgemäßen kosmetischen Zusammensetzungen enthalten bezogen auf das Gesamtgewicht der kosmetischen Zusammensetzungen 0,1 bis 20 Gew.-%, besonders bevorzugt 0,5 bis 10 Gew.-% des oben beschriebenen Vinylpyrrolidon-Polymers.

Das bevorzugte relative Gewichtsverhältnis von Polyurethan zu Vinylpyrrolidon-Polymer beträgt bevorzugt 1 zu 10 bis 10 zu 1, besonders bevorzugt 1 zu 5 bis 5 zu 1 und ganz besonders bevorzugt 1 zu 2 bis 2 zu 1.

Die erfindungsgemäßen kosmetischen Zusammensetzungen können neben den oben beschriebenen Polyurethanen II) und Vinylpyrrolidon-Polymeren I) weitere geeignete Filmbildner enthalten, welche insbesondere auch zur Festigung und zum Styling der Haare beitragen können.

Die Konzentration der weiteren Filmbildner kann von 0 bis 20 Gew.-% und bevorzugt 0 bis 10 Gew.-% jeweils bezogen auf den Festgehalt der Zusammensetzung betragen.

Vorteilhaft werden der oder die Filmbildner gewählt aus der Gruppe der von den erfindungsgemäß verwendeten Polyurethane verschiedenen wasserlöslichen oder wasserdispergierbaren Polyurethane, der Polyharnstoffe, Silikonharze und/oder Polyester sowie der nichtionischen, anionischen, amphoteren und/oder kationischen Polymere und ihren Mischungen.

Vorteilhafte nichtionische Polymere, die in erfindungsgemäßen Zusammensetzungen alleine oder im Gemisch, vorzugsweise auch mit anionischen und/oder amphoteren und/oder zwitterionischen Polymeren enthalten sein können, sind ausgewählt aus:
- Polyalkyloxazoline,
- Vinylacetat Homo- oder Copolymerisate. Hierzu gehören beispielweise Copolymerisate aus Vinylacetat und Acrylsäureester, Copolymerisate aus Vinylacetat und Ethylen, Copolymerisate aus Vinylacetat und Maleinsäureester,
- Acrylsäureester Copolymerisate wie z. B. die Copolymerisate aus Alkyl-Acrylat und Alkyl-Methacrylat, Copolymerisate aus Alkyl-Acrylat und Urethanen,
- Copolymerisate aus Acrylnitril und nichtionischem monomer ausgewählt aus Butadien und (Meth)Acrylat,
- Styrol Homo- und Copolymerisate. Hierzu gehören beispielweise Homopolystyrol, Copolymerisate aus Styrol und Alkyl-(Meth)Acrylat , Coplymerisate aus Styrol, AlkylMethacrylat und Alkyl-Acrylat, Copolymerisate aus Styrol und Butadien, Copolymerisate aus Styrol, Butadien und Vinylpyridin,
- Polyamide,
- Polyvinylcaprolactam, Polyvinylamide und deren Salze sowie Copolymere aus Dimethylaminoethyl-methacrylat,
- Polysiloxane,
- Homopolymere des N-Vinylformamids z. B. PVF von National Starch.

Besondere bevorzugte nichtionische Polymere sind Acrylsäureester Copolymerisate und, Polyvinylcaprolactam.

Vorteilhafte anionische Polymere sind Homo-oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbare-Verbindungen, welche mindestens eine Säuregruppe besitzen, insbesondere Carbonsäure, Sulfonsäure oder Phosphonsäure.

Vorteilhafte anionische Polymere enthaltend Carbonsäuregruppe sind:
- Acrylsäure oder Methacrylsäure Homo- oder Copolymer oder die Salze davon. Hierzu gehören beispielweise die Copolymerisate aus Acrylsäure und Acrylamide und/oder deren Natriumsalze, Copolymerisate aus Acrylsäure und/oder Methacrylsäure und einem ungesättigten Monomer ausgewählt aus Ethylene, Styrol, Vinylester, Acrylsäureester, Methacrylsäureester, gegebenenfalls ethoxylierten Verbindungen, Copolymerisate aus Methacrylsäure, Ethylacrylate und Tert-butyl Acrylate z.B. Luvimer® 100 P der Firma BASF.
- Crotonsäurederivat-Homo- oder Copolymer oder die Salze davon. Hierzu gehören beispielweise Vinylacetat/Crotonsäure-, Vinylacetat/Acrylat- und/oder Vinylacetat/Vinylneodecanoat/Crotonsäure-Copolymere, Natriumacrylat/Vinylalkohol-Copolymere,
- ungesättigte C4-C8 Carbonsäurederivate oder Carbonsäureanhydrid Copolymer ausgewählt aus Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester oder Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Allylester, Methallylester und ggfs. Acrylamide, Methacrylamide, alpha-Olefin, Acrylsäureester, Methacrylsäureester. Weitere bevorzugte Polymere sind Methylvinylether/MaleinsäureCopolymere, die durch Hydrolyse von Vinylether/Maleinsäureanhydrid-Copolymeren entstehen. Diese Polymere können auch teilverestert (Ethyl, Isopropyl- bzw. Butylester) oder teilamidiert sein.
- in Wasser lösliche oder dispergierbare anionische Polyurethane, z. B. Luviset ^{®}PUR von BASF, die von den erfindungsgemäßen Polyurethanen verschieden sind,
wobei diese Aufstellung selbstverständlich nicht limitierend sein soll.

Vorteilhafte anionische Polymere enthaltend Sulfonsäuregruppe sind Salze von Polyvinylsulfonsäure, Salze von Polystyrolsulfonsäure wie z. B. Natriumpolystyrolsulfonat oder Salze von Polyacrylamidesulfonsäure.

Besonders vorteilhafte anionische Polymere sind Acrylsäure Copolymere, Crotonsäurederivat-Copolymer, Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester und Salze von Polystyrolsulfonsäure.

Ganz besondere vorteilhafte anionische Polymere sind Acrylat-Copolymere, z.B. Luvimer von BASF, Ethylacrylat/N-tert.-Butylacrylamid/Acrylsäure-Copolymere ULTRAHOLD^{®} STRONG der Firma BASF, VA/Crotonat/Vinytneodecanoat-Copolymer z. B. Resyn 28-2930 von National Starch, Copolymerisate wie. Z. Copolymerisate aus Methylvinylether und Maleinsäureanhydrid teilverestert z.B. GANTREZ^{®} der Firma ISP und Natrium-Polystyrol-Sulfonate z. B. Flexan 130 von National Starch.

Vorteilhafte amphotere Polymere können unter den Polymeren ausgewählt werden, die statistisch in der Polymerkette verteilte Einheiten A und B enthalten, wobei A eine Einheit bedeutet, die von einem Monomer mit mindestens einem basischen Stickstoffatom abgeleitet ist, und B eine Einheit ist, die von einem sauren Monomer stammt, das eine oder mehrere Carboxygruppen oder Sulfonsäuregruppen aufweist, oder A und B können Gruppen bedeuten, die von zwitterionischen Carboxybetainmonomeren oder Sulfobetainmonomeren abgeleitet sind; A und B können auch eine kationische Polymerkette bedeuten, die primäre, sekundäre, tertiäre oder quartanäre Gruppen enthält, worin mindestens eine Aminogruppe eine Carboxygruppe oder Sulfonsäuregruppe trägt, die über eine Kohlenwasserstoffgruppe gebunden ist, oder B und C sind Teil einer Polymerkette mit Ethylen-α,β-diearbonsäureeinheit, bei der die Carbonsäuregruppen mit einem Polyamin umgesetzt wurden, das eine oder mehrere primäre oder sekundäre Aminogruppen enthält.

Besondere vorteilhafte amphote Polymere sind:
- Polymere, die bei der Copolymerisation eines von einer Vinylverbindung mit Carboxygruppe abgeleiteten Monomers, wie insbesondere Acrylsäure, Methacrylsäure, Maleinsäure, α-Chloracrylsäure, und einem basischen Monomer gebildet werden, das von einer Vinylverbindung abgeleitet ist, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent Nr. 3 836 537 beschrieben worden.
- Polymere mit Einheiten, die abgeleitet sind von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat.
   Erfindungsgemäß besonders bevorzugte N-substituierte Acrylamide oder Methacrylamide sind Verbindungen, deren Alkylgruppen 2 bis 12 Kohlenstoffatome enthalten, besonders N-Ethylacrylamid, N-t-Butylacrylamid, N-t-Octylacrylamid, N-Octylacrylamid, N-Decylacrylamid, N-Dodecylacrylamid sowie die entsprechenden Methacrylamide.
   Die sauren Comonomere sind insbesondere unter Acrylsäure, Methacrylsäure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.
   Bevorzugte basische Comonomere sind Aminoethylmethacrylat, Butylaminoethylmethacrylat, N,N-Dimethylaminoethylmethacrylat, N-t-Butylaminoethylmethacrylat.
- Vernetzte und ganz oder teilweise acylierte Polyaminoamide, die von Polyaminoamiden der folgenden allgemeinen Formel abgeleitet sind:

   -[CO-R-CO-Z]-
worin R eine zweiwertige Gruppe bedeutet, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bissekundäres Amin entsteht, und Z eine Gruppe bedeutet, die von einem bis-primären, mono- oder bis-sekundären Polyalkylenpolyamin abgeleitet ist, und vorzugsweise: a) in Mengenanteilen von 60 bis 100 Mol-% die Gruppen -NH-[(CH₂)ₓ-NH-]ₚ- mit x = 2 und p = 2 oder 3 oder x = 3 und p = 2, wobei diese Gruppe, von Diethylentriamin, Triethylentetramin oder Dipropylentriamin abgeleitet ist; b) in Mengenanteilen von 0 bis 40 Mol% die Gruppe -NH-[(CH₂)ₓ-NH-]ₚ-, worin x = 2 und p = 1, die von Ethylendiamin abgeleitet ist, oder die Gruppe, die von Piperazin stammt: 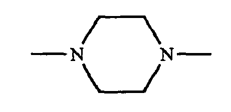 c) in Mengenanteilen von 0 bis 20 Mol%, die Gruppe -H-(CH₂)₆-NH-, die von Hexamethylendiamin abgeleitet ist, wobei diese Polyaminoamide durch Addition eines bifunktionellen Vernetzungsmittels, das unter den Epihalohydrinen, Diepoxiden, Dianhydriden und bis-ungesättigten Derivaten ausgewählt ist, in einer Menge von 0,025 bis 0,35 mol Vernetzungsmittel pro Aminogruppe des Polyaminoamids vernetzt und mit Acrylsäure, Chloressigsäure oder einem Alkansulton oder deren Salzen acyliert ist.

Die gesättigten Carbonsäuren sind vorzugsweise unter den Säuren mit 6 bis 10 Kohlenstoffatomen ausgewählt, wie Adipinsäure, 2,2,4-Trimethyladipinsäure und 2,4,4,-Trimethyladipinsäure, Terephthalsäure; Säuren mit ethylenischer Doppelbindung, wie beispielsweise Acrylsäure, Methacrylsäure und Itaconsäure.

Die bei der Acylierung verwendeten Alkansultone sind vorzugsweise Propansulton oder Butansulton, die Salze der Acylierungsmittel sind vorzugsweise die Natriumsalze oder Kaliumsalze.
- Polymeren mit zwitterionischen Einheiten der folgenden Formel: 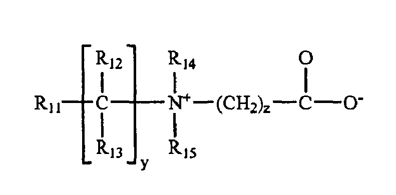 worin R₁₁ eine polymerisierbare ungesättigte Gruppe bedeutet, wie Acrylat, Methacrylat, Acrylamid oder Methacrylamid, y und z ganze Zahlen von 1 bis 3 bedeuten, R₁₂ und R₁₃ ein Wasserstoffatom, Methyl, Ethyl oder Propyl bedeuten, R₁₄ und R₁₅ ein Wasserstoffatom oder eine Alkylgruppe bedeuten, die so gewählt ist, dass die Summe der Kohlenstoffatome R₁₄ und R₁₅ 10 nicht übersteigt.
   Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.
- Polymere, die von Chitosan abgeleitet sind und Monomereinheiten enthalten, die den folgenden Formeln entsprechen: 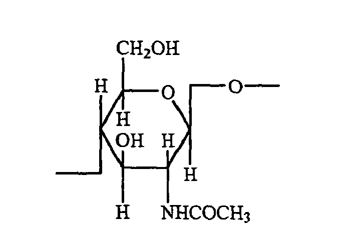 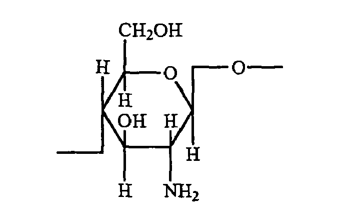 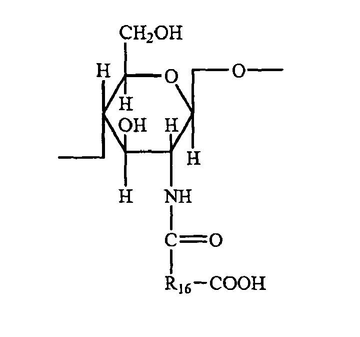
wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit R₁₆ eine Gruppe der folgenden Formel bedeutet: 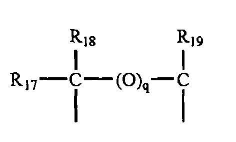 worin bedeuten: falls q = 0, die Gruppen R₁₇, R₁₈ und R₁₉, die gleich oder verschieden sind, jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen und/oder gegebenenfalls mit einer oder mehreren Gruppen Amino, Hydroxy, Carboxy, Alkylthio, Sulfonsäure, Alkylthio, dessen Alkylgruppe einen Aminorest trägt, wobei mindestens eine der Gruppen R₁₇, R₁₈ und R₁₉ in diesem Fall ein Wasserstoffatom bedeutet; oder falls q = 1, die Gruppen R₁₇, R₁₈ und R₁₉ jeweils ein Wasserstoffatom, sowie die Salze, die diese Verbindungen mit Basen oder Säuren bilden.
- Polymere, die der folgenden allgemeinen Formel entsprechen und die beispielsweise in dem französischen Patent 1 400 366 beschrieben sind: 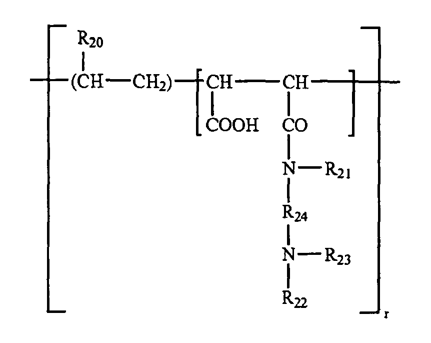
worin R₂₀ ein Wasserstoffatom, CH₃₀, CH₂CH₂O oder Phenyl bedeutet, R₂₁ ein Wasserstoffatom oder eine niedere Alkylgruppe, wie Methyl oder Ethyl ist, R₂₂ ein Wasserstoffatom oder eine niedere C₁₋₆-Alkylgruppe bedeutet, wie Methyl oder Ethyl, R₂₃ eine niedere C₁₋₆-Alkylgruppe ist, wie Methyl oder Ethyl oder eine Gruppe der Formel: -R₂₄-N(R₂₂)₂, wobei R₂₄ eine Gruppe -CH₂-CH₂, -CH₂-CH₂-CH₂- oder -CH₂-CH(CH₃)- bedeutet und wobei R₂₂ die oben angegebenen Bedeutungen aufweist.
- Polymere, die bei der N-Carboxyalkylierung von Chitosan gebildet werden können, wie N-Carboxymethylchitosan oder N-Carboxybutylchitosan.
- Amphotere Polymere vom Typ -D-X-D-X, die ausgewählt sind unter:
   a) Polymere, die durch Einwirkung von Chloressigsäure oder Natriumchloracetat auf Verbindungen mit mindestens einer Einheit der folgenden Formel gebildet werden:
      worin D die Gruppe bedeutet 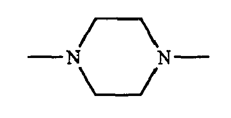 und X die Symbole E oder E' bedeutet, wobei E oder E', die gleich oder verschieden sind, eine zweiwertige Gruppe bedeuten, bei der es sich um eine geradkettige oder verzweigte Alkylengruppe mit bis zu 7 Kohlenstoffatomen in der Hauptkette handelt, die unsubstituiert vorliegt oder mit Hydroxygruppen substituiert ist und ein oder mehrere Sauerstoffatome, Stickstoffatome oder Schwefelatome und 1 bis 3 aromatische und/oder heterocyclische Ringe enthalten kann; wobei die Sauerstoffatome, Stickstoffatome und Schwefelatome in Form der folgenden Gruppen vorliegen: Ether, Thioether, Sulfoxid, Sulfon, Sulfonium, Alkylamin, Alkenylamin, Hydroxy, Benzylamin, Aminoxid, quartäres Ammonium, Amid, Imid, Alkohol, Ester und/oder Urethan.
   b) Polymere der Formel-D-X-D-X worin D die Gruppe bedeutet 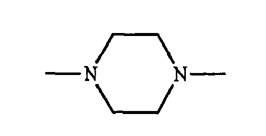 und X das Symbol E oder E' und mindestens einmal E' bedeutet; wobei E die oben angegebenen Bedeutungen aufweist und E' eine zweiwertige Gruppe ist, bei der es sich um eine geradkettige oder verzweigte Alkylengruppe mit bis zu 7 Kohlenstoffatomen in der Hauptkette handelt, die unsubstituiert vorliegt oder mit einer oder mehreren Hydroxygruppen substituiert ist und ein oder mehrere Stickstoffatome enthält, wobei das Stickstoffatom mit einer Alkylgruppe substituiert ist, die gegebenenfalls durch ein Sauerstoffatom unterbrochen ist und zwingend eine oder mehrere Carboxyfunktionen oder eine oder mehrere Hydroxyfunktionen enthält und durch Umsetzung mit Chloressigsäure oder Natriumchloracetat betainisiert ist.
- Alkyl(C₁₋₅)vinylether/Maleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol teilweise modifiziert sind. Diese Polymere können auch weitere Comonomere enthalten, wie Vinylcaprolactam.

Ganz besondere vorteilhafte amphotere Polymere sind z.B. die Copolymere Octylacrylamid/AcrylateButylamino-Ethylmethacrylat-Copolymer, die unter den Bezeichnungen AMPHOMER^{®}, AMPHOMER^{®} LV 71 oder BALANCE^{®} 47 der Firma NATIONAL STARCH im Handel sind, und MethylmethacryLat/Methyl-dimethylcarboxymethylammoniumethylmethacrylat-Copolymere.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren.

Als Neutralisationsmittel für Polymere die Säuregruppen enthalten können folgende Basen eingesetzt werden: Hydroxide, deren Kation ein Ammonium oder ein Alkalimetall ist wie z. B. NaOH oder KOH.

Andere Neutralisationsmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS).

Die Neutralisation kann je nach Anwendungszweck teilweise oder vollständig erfolgen.

Gegebenfalls einsetzbar aber jedoch weniger bevorzugt sind kationische Polymere, wie beispielweise Polymere, die primäre, sekundäre tertiäre und/oder quaternäre Aminogruppen enthalten, die Teil der Polymerkette oder direkt an die Polymerkette gebunden sind.

Das kosmetische akzeptable Medium enthält insbesondere Wasser und gegebenenfalls ein kosmetisch geeignetes Lösungsmittel. Die bevorzugten Lösungsmittel sind aliphatische Alkohole mit C2-4 Kohlenstoffatomen wie Ethanol, Isopropanol, t-Butanol, n-Butanol; Polyol wie Propylenglycol, Glycerin, Ethylenglycol und Polyolethern; Aceton; unverzweigte oder verzweigte Kohlenwasserstoffe wie Pentan, Hexan, Isopentan und zyklische Kohlenwasserstoffe wie Cyclopentan und Cyclohexan; und deren Gemischen ausgewählt.

Ganz besonderes bevorzugtes Lösungsmittel ist Ethanol.

Der Gehalt an derartigen Lösungsmitteln liegt aber entsprechend der Tatsache, dass es sich erfindungsgemäß bevorzugt um VOC-arme Haarfestiger-Zusammensetzungen handelt, bevorzugt bei weniger als 80 Gew.-%, noch bevorzugter bei weniger als 55 Gew.-%, noch bevorzugter bei weniger als 40 Gew.-%.

Der Wasseranteil kann insbesondere im Bereich beispielsweise von 20 bis 94 Gew.-%, bevorzugt von 30 bis 80 Gew.-%, noch bevorzugter von mehr als 45 bis 70 Gew.-%, bezogen auf das Gesamtgewicht der Zusammensetzung, liegen. Das Medium ist vorteilhaft ein wässrig-alkoholisches Gemisch. Der Mengenanteil des Alkohols im Gemisch liegt im Bereich von 0 bis 90 Gew.-%, vorzugsweise 0 bis 70 Gew.-% bevorzugter 0 bis 55 Gew.%, noch bevorzugter bei 0 bis 40 Gew.-%, bezogen auf das Gesamtgewicht der Zusammensetzung.

Die erfindungsgemäßen kosmetischen Zusammensetzungen können insbesondere bei Verwendung als Haarfestiger des weiteren Verdicker enthalten.

Vorteilhafte Verdicker sind:
- Vernetzte oder nichtvernetzte Acrylsäure oder Methacrylsäure Homo- oder Copolymere. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate und Vinylacetat.
- Verdickende Polymere natürlicher Herkunft beispielweise auf Cellulosebasis, Guargummi, Xanthan, Scleroglucan, Gellangummi, Rhamsan und Karayagummi, Alginate, Malto-dextrin, Stärke und ihre Derivate, Johannisbrotkernmehl, Hyaluronsäure.
- Nichtionische, anionische, kationische oder amphotere assoziative Polymere z.B. auf Basis von Polyethylenglycole und ihre Derivate, oder Polyurethane.
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure angegeben werden.

Besondere vorteilhafte Verdicker sind verdickende Polymere natürlicher Herkunft, vernetzte Acrylsäure oder Methacrylsäure Homo- oder Copolymere und vernetzte Copolymere von 2-Acrylamido-2-methylpropansulfonsäure.

Ganz besonders bevorzugte Verdicker sind Xanthangummi, wie die unter den Bezeichnungen Keltrol^{®} und Kelza^{®} von der Firma CP Kelco angebotenen Produkte oder die Produkte der Firma, RHODIA mit der Bezeichnung Rhodopol und Guargummi, wie die unter der Bezeichnung Jaguar^{®} HP105 von der Firma RHODIA erhältlichen Produkte.

Ebenfalls ganz besonders bevorzugte Verdicker sind vernetzte Homopolymere von Methacrylsäure oder Acrylsäure, die von der Firma Lubrizol unter den Bezeichnungen Carbopol^{®} 940, Carbopol^{®} 941, Carbopol^{®} 980, Carbopol^{®} 981, Carbopol^{®} ETD 2001, Carbopol^{®} EDT 2050, Carbopol^{®} 2984, Carbopol^{®} 5984 und Carbopol^{®} Ultrez 10, von der Firma 3V unter den Bezeichnungen Synthalen^{®} K, Synthalen^{®} L und Synthalen^{®} MS und von der Firma PROTEX unter den Bezeichnungen Modarez^{®} V 1250 PX, Modarez^{®} V2000 PX, Viscaron^{®} A1600 PE und Viscaron^{®} A700 PE im Handel erhältlich sind.

Ebenfalls ganz besonders bevorzugte Verdicker sind vernetzte Copolymere von Acrylsäure oder Methacrylsäure und einem C₁₀₋₃₀-Alkylacrylat oder C₁₀₋₃₀-ALkylmethacrylat und Copolymere von Acrylsäure oder Methacrylasäure und Vinylpyrrolidon. Solche Copolymere sind beispielsweise von der Firma Lubrizol unter den Bezeichnungen Carbopol^{®} 1342, Carbopol^{®} 1382, Pemulen^{®} TR1 oder Pemulen^{®} TR2 und von der Firma ISP unter den Bezeichnungen Ultrathix P-100 (INCI: Acrylic Acid/VP Crosspolymer) im Handel erhältlich.

Ebenfalls ganz besonders bevorzugte Verdicker sind vernetzte Copolymere von 2-Acrylamido-2-methylpropansulfonsäure. Solche Copolymere sind beispielsweise von der Firma Clariant unter den Bezeichnungen Aristoflex^{®} AVC (INCI: Ammonium Acryloyldimethyltaurat/VP Copolymer) erhältlich

Falls Verdicker verwendet werden, sind sie im Allgemeinen in einer Konzentration von 0,01 % bis 2 Gew.-% vorzugsweise 0,1 % bis 1 Gew.-% bezogen auf das Gesamtgewicht der Zusammensetzung vorhanden.

Die erfindungsgemäßen kosmetischen Zusammensetzungen können insbesondere bei Verwendung als Haarfestiger des weiteren ein Treibgas enthalten. Dabei ist es vorteilhaft, das Treibgas in einer Menge von 1 bis 40 Gewichts-% und besonders bevorzugt in einer Konzentration von 5 bis 20 Gewichts-% bezogen auf das Gesamtgewicht der Formulierung einzusetzen.

Die erfindungsgemäßen bevorzugten Treibgase sind Kohlenwasserstoffe wie Propan, Isobutan und n-Butan sowie deren Mischungen und Dimethylether. Aber auch Druckluft, Kohlendioxid, Stickstoff, Stickstoffdioxid sowie Mischungen all dieser Gase sind erfindungsgemäß vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW) wie z.B. 1,2-difluoroethan (Treibmittel 152 A).

In den erfindungsgemäßen kosmetischen Zusammensetzungen können insbesondere bei Verwendung als Haarfestiger des weiteren haarpflegende Wirkstoffe verwendet werden.

Als solche Pflegestoffe können bevorzugt cyclische Polydimethylsiloxane (Cyclomethicone) in Konzentrationen von z. B. 0-1,0 Gew.-% der Gesamtformulierung oder Silikontenside (Polyethermodifizierte Siloxane) vom Typ Dimethicone Copolyol oder Simethicon z. B. in Konzentrationen von 0 bis 1,0 Gew.-% des Gesamtgewichts der Zusammensetzung bevorzugt zum Einsatz kommen. Cyclomethicone werden u. a. unter der Handelsbezeichnungen Abil^{®} K4 von der Firma Goldschmidt oder z.B. DC 244, DC 245 oder DC 345 von der Firma Dow Corning angeboten. Dimethicon-Copolyole werden z. B. unter der Handelsbezeichnung DC 193 von der Firma Dow Corning bzw. Belsil^{®} DM 6031 von der Firma Wacker angeboten.

Optional können herkömmliche Additive ebenfalls in den erfindungsgemäßen kosmetischen Zusammensetzungen insbesondere bei Verwendung als Haarfestiger enthalten sein, beispielsweise um der Zusammensetzung bestimmte Modifizierungseigenschaften zu verleihen: dies sind Silikone oder Silikonderivate, Benetzungsmittel, Feuchthaltemittel, Weichmacher wie Glycerin, Glykol und Phthalester und -ether, Riechstoffe und Parfüms, UV-Absorber, Farbstoffe, Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Tenside, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner.

Diese Additive sind im Allgemeinen in einer Konzentration von etwa 0,001 % bis 15 Gew.% vorzugsweise 0,01 % bis 10 Gew.-% bezogen auf das Gesamtgewicht der erfindungsgemäßen kosmetischen Zusammensetzungen vorhanden.

Vorteilhaft können die erfindungsgemäßen kosmetischen Zusammensetzungen insbesondere bei Verwendung als Haarfestiger in Form eines Sprays, eines Schaums, eines Gels, einer Emulsion, einer Lösung oder einer Creme vorliegen, wie als Schaumfestiger, Flüssigfestiger, Haarspray, Stylinggel, Stylingcreme, Schaumaerosol etc..

Die erfindungsgemäßen kosmetischen Zusammensetzungen können daher vorteilhafter Weise in einer Pumpspray- oder Aerosolverpackung vorliegen.

Eine Aufschäumung erfolgt bevorzugt mit einem Treibgas. Dementsprechend sind Pumpspray-, Aerosolverpackungs und Schaumspender auf Pumpspray- oder Aerosolverpackungsbasis, welche die erfindungsgemäßen Zusammensetzungen enthalten, ebenfalls Gegenstand der Erfindung.

Eine bevorzugte Ausführungsform der erfindungsgemäßen Zusammensetzungen liegt in der Form eines Mousse vor, welches zusätzlich einen oder mehrere der folgenden Bestandteile enthält: kosmetisch geeignete Lösungsmittel, wie aliphatische Alkohole mit 2-4 Kohlenstoffatomen, bevorzugt Ethanol, Polyole, Aceton, unverzweigte oder verzweigte Kohlenwasserstoffe, zyklische Kohlenwasserstoffe und deren Gemische, sowie Treibgase wie Kohlenwasserstoffe, Druckluft, Kohlendioxid, Stickstoff, Stickstoffdioxid, Dimethylether, Fluorkohlenwasserstoffe und Fluorchlorkohlenwasserstoffe, bevorzugt Dimethylether und/oder ein Propan/Butan-Gemisch.

Eine weitere bevorzugte Ausführungsform der erfindungsgemäßen Zusammensetzungen liegt in der Form eines Gels vor, welches zusätzlich einen oder mehrere der folgenden Bestandteile enthält: kosmetisch geeignete Lösungsmittel, wie Wasser und/oder aliphatische Alkohole mit 2-4 Kohlenstoffatomen, bevorzugt Ethanol, Polyole, Aceton, unverzweigte oder verzweigte Kohlenwasserstoffe, zyklische Kohlenwasserstoffe und deren Gemische, sowie einen Verdicker, bevorzugt verdickende Polymere natürlicher Herkunft und/oder vernetzte oder nicht vernetzte Acrylsäure Homo- oder Copolymere.

### Beispiele:

Alle Mengenangaben, Anteile und Prozentanteile sind, soweit nicht anders angegeben, auf das Gewicht und die Gesamtmenge bzw. auf das Gesamtgewicht der Zusammensetzungen bezogen.

Sofern nicht abweichend vermerkt, beziehen sich alle analytischen Messungen auf Messungen bei Temperaturen von 23°C.

Die Feststoff bzw. Festkörpergehalte werden ermittelt durch Erhitzen einer ausgewogenen Probe auf 125°C bis zur Gewichtskonstanz. Bei Gewichtskonstanz wird durch erneutes Auswiegen der Probe der Festkörpergehalt berechnet.

NCO-Gehalte wurden, wenn nicht ausdrücklich anders erwähnt, volumetrisch gemäß DIN-EN ISO 11909 bestimmt.

Die Kontrolle auf freie NCO-Gruppen wurde mittels IR-Spektroskopie (Bande bei 2260 cm⁻¹) durchgeführt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

### Verwendete Substanzen und Abkürzungen:

- Diaminosulfonat:: NH₂-CH₂CH₂-NH-CH₂CH₂-SO₃Na (45 %ig in Wasser)
- Desmophen^{®} 2020/C2200:: Polycarbonatpolyol, OH-Zahl 56 mg KOH/g, zahlenmittleres Molekulargewicht 2000 g/mol (Bayer MaterialScience AG, Leverkusen, DE)
- PolyTHF^{®} 2000:: Polytetramethylenglykolpolyol, OH-Zahl 56 mg KOH/g, zahlenmittleres Molekulargewicht 2000 g/mol (BASF AG, Ludwigshafen, DE)
- PolyTHF^{®} 1000:: Polytetramethylenglykolpolyol, OH-Zahl 112 mg KOH/g, zahlenmittleres Molekulargewicht 1000 g/mol (BASF AG, Ludwigshafen, DE)
- Polyether LB 25:: monofunktioneller Polyether auf Ethylenoxid-/Propylenoxidbasis zahlenmittleres Molekulargewicht 2250 g/mol, OH-Zahl 25 mg KOH/g (Bayer MaterialScience AG, Leverkusen, DE)

### Beispiel 1: Polyurethan-Dispersion 1

987,0 g PolyTHF^{®} 2000 (Komponente A2)), 375,4 g PolyTHF^{®} 1000 (Komponente A2)), 761,3 g Desmophen^{®} C2200 (Komponente A2)) und 44,3 g Polyether LB 25 (Komponente A4)) wurden in einer Standard-Rührapparatur auf 70°C aufgeheizt. Anschließend wurde ein Gemisch aus 237,0 g Hexamethylendiisocyanat (Komponente A1)) und 313,2 g Isophorondiisocyanat (Komponente A1)) zugegeben und bei 120°C gerührt, bis der theoretische NCO-Wert erreicht war. Das fertige Prepolymer wurde mit 4830 g Aceton gelöst und dabei auf 50°C abgekühlt und anschließend wurde eine Lösung aus 25,1 g Ethylendiamin (Komponente B1)), 116,5 g Isophorondiamin (Komponente B1)), 61,7 g Diaminosulfonat (Komponente B2)) und 1030 g Wasser zudosiert. Die Nachrührzeit betrug 10 min. Dann wurde durch Zugabe von 1250 g Wasser dispergiert. Es folgte die Entfernung des Lösemittels durch Destillation im Vakuum.

Die erhaltene weiße Dispersion hatte nachfolgende Eigenschaften:

| | |
|---|---|
| Feststoffgehalt: | 61% |
| Partikelgröße (LKS): | 312 nm |
| Viskosität (Viskosimeter, 23°C): | 241 mPas |
| pH (23°C): | 6,02 |

### Beispiel 2: Polyurethan-Dispersion 2

450 g PolyTHF^{®} 1000 (Komponente A2)) und 2100 g PolyTHF^{®} 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 29,5 g Ethylendiamin (Komponente B1)), 143,2 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 56 % |
| Partikelgröße (LKS): | 276 nm |
| Viskosität: | 1000 mPas |

### Beispiel 3: Polyurethan-Dispersion 3

1649,0 g eines Polyesters aus Adipinsäure, Hexandiol und Neopentylglykol mit einem mittleren Molekulargewicht von 1700 g/mol (Komponente A2)) wurden auf 65°C aufgeheizt. Anschließend wurden 291,7 g Hexamethylendiisocyanat (Komponente A1)) zugegeben und solange bei 100-115°C gerührt bis der theoretische NCO-Wert unterschritten war. Das fertige Prepolymer wurde mit 3450 g Aceton bei 50°C gelöst und anschließend eine Lösung aus 16,8 g Ethylendiamin (Komponente B1)), 109,7 g Diaminosulfonat (Komponente B2)) und 425 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42 % |
| Partikelgröße (LKS): | 168 nm |
| Viskosität: | 425 mPas |
| pH-Wert: | 7,07 |

### Beispiel 4: Polyurethan-Dispersion 4

340 g eines Polyesters aus Adipinsäure, Hexandiol und Neopentylglykol mit einem mittleren Molekulargewicht von 1700 g/mol (Komponente A2)) wurden auf 65°C aufgeheizt. Anschließend wurden 60,1 g Hexamethylendiisocyanat (Komponente A1)) zugegeben und solange bei 105°C gerührt bis der theoretische NCO-Wert unterschritten war. Das fertige Prepolymer wurde mit 711 g Aceton bei 50°C gelöst und anschließend eine Lösung aus 2,1 g Ethylendiamin (Komponente B1)), 32,4 g Diaminosulfonat (Komponente B2)) und 104,3 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 40 % |
| Partikelgröße (LKS): | 198 nm |
| Viskosität: | 700 mPas |
| pH-Wert: | 6,31 |

### Beispiel 5: Polyurethan-Dispersion 5

450 g PolyTHF^{®} 1000 (Komponente A2)) und 2100 g PolyTHF^{®} 2000 (Komponente A2)) wurden auf 70°C aufgeheizt. Anschließend wurde ein Gemisch aus 225,8 g Hexamethylendiisocyanat (Komponente A1)) und 298,4 g Isophorondiisocyanat (Komponente A1)) zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 351 g Diaminosulfonat (Komponente B2)) und 610 g Wasser zudosiert. Die Nachrührzeit betrug 15 min. Danach wurde durch Zugabe von 1880 g Wasser dispergiert. Es folgte die Entfernung des Lösemittels durch Destillation im Vakuum und es wurde eine lagerstabile Dispersion erhalten.

| | |
|---|---|
| Feststoffgehalt: | 42 % |
| Viskosität: | 1370 mPas |

### Anwendungstechnische Vergleichversuche:

| Versuch | A | B | C |
|---|---|---|---|
| Carbomer (gew.%), Carbopol 910 (Noveon) | 0,35 | 0,35 | 0,35 |
| Polyurethan (Gew.% Feststoff), Polyurethanedispersion aus dem Beispiel 3 | 2 | - | 2 |
| PVP-K90 (Luviskol K90, -BASF) | - | 2 | 2 |
| Wasser (Gew.%) | ad 100 | ad 100 | ad 100 |

Die mechanischen Eigenschaften werden mittels des sogenannten Tests " Omega Loop" gemessen. Für die Omega-Loop Versuche werden kommerziell Mischhaare der Firma International Hair Importers, New York. (Gewicht: 2 g, Nutzlänge: 16,5 cm, Breite: 3,2 cm) verwendet. Die Haare werden vor der Anwendung einer standardisierten Waschprozedur unterzogen. Die im Wasser eingeweichten Haare werden mit 3 Gew.-% Ammoniumlaurylsulfatlösung zwei Minuten shampooniert, gründlich mit warmen Wasser ausgespült, kalt trocken geföhnt und bei 22°C und 55% relativer Feuchtigkeit konditioniert. 0,15 g Formulierung pro 0,2 g Haar werden mittels eines Spatels homogen auf die Haare verteilt.

Die "Omega-Loop" Versuche werden wie in der Literatur beschrieben durchgeführt. (J. Jachowicz und K. Yao, Dynamic Hairspray Analysis I. Instrumentation and Preliminary Results. J. Soc. Cosmet.Chem., 47, 73 (1996)). Der Omega Loop Versuche werden mittels eines Texture Analyzer (Model TA-XT2) von der Firma Texture Technologies Corporation durchgeführt.

Gemessen werden:
F1: maximale Kraft nach der ersten Deformation (entspricht das Haltvermögen)
E10/E1 : Verhältnis zwischen der Steigung nach der 10. Deformation zu der Steigung nach der ersten Deformation, das die Flexibilität des Polymerfilms auf die Haare entspricht.

| Versuch | A | B | C |
|---|---|---|---|
| F1 | 374,7± 37,2 | 469,8± 18,4 | 650,4± 54,9 |
| E10/E1 | 0,28±0,02 | 0,30±0,02 | 0,44 ± 0,03 |

Die Versuche zeigen deutlich, das die Kombination eines Polyvinylpyrrolidons mit dem erfindungsgemäß verwendeten Polyurethan einen flexiblen Polymerfilm (E10/E1 Werte) auf die Haare im vergleich zu dem einzelnen Polymer erzielt. Die Formulierung enthaltend die Polymerkombination zeigt ein stärkeres Haltvermögen (F1 Werte) als die Formulierungen mit einzeln Filmbildnerpolymer.

Die Schuppenbildung wird mittels Bilderanalyse von mit den oben genannten Formulierungen behandelten Haarsträhnen bestimmt. Die Schuppen werden durch die Anzahl von lichtgestreuten Stellen quantifiziert.

| Versuch | A | B | C |
|---|---|---|---|
| Anzahl von lichtgestreuten Stellen | 48 | 43 | 36 |

Durch die erfindungsgemäße Kombination verringert sich die Anzahl an Schuppen in überraschender Weise.

Die Haptik der Haarsträhne wird durch ein Panel von drei Personen evaluiert. Eine Note zwischen 1 (schlecht) bis 5 (gut) wird gegeben.

| Versuch | B | C |
|---|---|---|
| Haptik | 1,7 | 3,3 |

Die Kombination aus PUR und PVP bewirkt eine Verbesserung.

### Anwendungstechnische Beispiele:

(Angegeben sind Gewichtsteile).
VP = Vinylpyrrolidion
VA = Vinylacetat
VA/VP Copolymer: Copolymer mit variablem Mengenbausteinen einsetzbar, Beispiele für die Gewichtsverhälntisse lauten: VP/VA : 30/70, 60/40, 50/50 oder 70/30.
"Pump-setting-Spray"

| | A | B |
|---|---|---|
| Erfindungsgemäßes Polyurethan (bezogen auf Feststoff) | 2 | 5 |
| Polvyinylpyrrolidon | 2 | |
| VP/VA copolymer (bezogen auf Feststoff) | | 5 |
| Ethanol | 55 | 30 |
| Parfüm | q.s. | q.s. |
| Wasser | ad 100 | ad 100 |

Aerosol-Hair-Spray

| | C | D | E | F | G |
|---|---|---|---|---|---|
| Erfindungsgemäßes Polyurethan (bezogen auf Feststoff) | 5 | 10 | 2 | 5 | 8 |
| Polvyinylpyrrolidon | 1 | | | 1,8 | |
| VP/VA copolymer (bezogen auf Feststoff) | | 5 2 | | | 5 |
| Aminomethylpropanol | | | q.s | q.s | |
| Glycerin | | 0,5 | | | |
| Panthenol | | | 0,5 | | 0,5 |
| PEG/PPG-18/18 Dimethicone | | | | 0,5 | |
| PEG-12 dimethicone | | 0,05 | | | |
| Propylene Glycol | | | | 0,5 | |
| Cyclomethicone | | | 1,0 | | 1,0 |
| Benzophenone-3 | | 0,1 | 0,1 | | 0,1 |
| Parfüm | q.s | q.s | q.s | q.s | q.s |
| Ethanol | 14,5 | 20 | 60 | 30 | 20 |
| Wasser | ad 100 | ad 100 | ad 100 | ad 100 | ad 100 |
| Propan/butan 3,5 Bar. (20°C) | | | 20 | 10 | |
| Dimethylether | 40 | 30 | | 30 | 20 |
| Fluorkohlenwasserstoff 152 A | | | | | 20 |

Haarschaum

| | H | I |
|---|---|---|
| Erfindungsgemäßes Polyurethan (bezogen auf Feststoff) | 2 | 4 |
| Polvyinylpyrrolidon | | 2 |
| VP/VA copolymer (bezogen auf Feststoff) | 5 | |
| Glycerin | 0,1 | |
| Panthenol | 0,05 | 0,5 |
| Polyquaternium-4 | 2 | |
| Cetyltrimethylammoniumchlorid | 0,2 | 0,5 |
| PEG-12 dimethicone | | 0,5 |
| Cyclomethicone | | 0,5 |
| Benzophenone-3 | 0,1 | |
| Parfüm | q.s. | q.s. |
| Ethanol | 15 | 10 |
| Wasser | ad 100 | ad 100 |
| Konservierungsmittel | q.s. | q.s. |
| Dimethylether | 10 | 7 |
| Fluorkohlenwasserstoff 152 A | | 3 |

Haargel/creme

| | J | K | L |
|---|---|---|---|
| Erfindungsgemäßes Polyurethan (bezogen auf Feststoff) | 5 | 2 | 8 |
| Polvyinylpyrrolidon | | 5 | |
| VP/VA copolymer (bezogen auf Feststoff) | 1 | | 2 |
| Carbomer | 0,8 | | |
| Acrylsäure/VP Copolymer | | 0,5 | |
| Ammonium acryloyldimethyltau-rat/VP Copolymer | | | 0,8 |
| Glycerin | 0,5 | | |
| Panthenol | | 0,5 | 0,5 |
| Propylenglycol | | | 0,2 |
| Cyclomethicone | | 0,2 | |
| Neutralisierungsmittel | q.s. | q.s. | q.s. |
| Parfüm | q.s. | q.s. | q.s. |
| Ethanol | 20 | | |
| Wasser | ad.100 | ad.100 | ad. 100 |
| Konservierungsmittel | q.s. | q.s. | q.s. |

## Patentansprüche

1. Kosmetische Zusammensetzungen, enthaltend eine Kombination von Vinylpyrrolidon-Copolymeren I) und Polyurethanen II), wobei die Polyurethane erhältlich sind durch Umsetzung eines oder mehrerer wasserunlöslicher, nicht wasserdispergierbarer, isocyanatfunktioneller Polyurethanprepolymere A) mit einer oder mehreren aminofunktionellen Verbindungen B).

2. Kosmetische Zusammensetzungen gemäß Anspruch 1, **dadurch gekennzeichnet, dass** die den Polyurethanen II) zugrundeliegenden Prepolymere A) eine Wasserlöslichkeit bei 23°C von weniger als 10 g / Liter und einen Gehalt an ionischen und ionogenen Gruppen von 15 Milliequivalenten pro 100 g Prepolymer A) aufweisen.

3. Kosmetische Zusammensetzungen gemäß Anspruch 1 oder 2, **dadurch gekennzeichnet, dass** die Polyurethane II) in Form wässriger Dispersionen bei der Herstellung der Zusammensetzungen eingesetzt werden.

4. Kosmetische Zusammensetzungen gemäß Anspruch 3, **dadurch gekennzeichnet, dass** die wässrigen Polyurethandispersionen erhältlich sind, in dem
A) isocyanatfunktionelle Prepolymere aus
A1) organischen Polyisocyanaten,
A2) polymeren Polyolen,
A3) gegebenenfalls hydroxyfunktionellen Verbindungen mit Molekulargewichten von bevorzugt 62 bis 399 g/mol, und
A4) gegebenenfalls nichtionischen Hydrophilierungsmitteln,
hergestellt werden, wobei die isocyanatfunktionellen Prepolymere A) einen Gehalt an ionischen und ionogenen Gruppen von weniger als 15 Milliequivalenten pro 100 g Prepolymer A) aufweisen, und
B) deren freie NCO-Gruppen dann ganz oder teilweise
mit einer oder mehreren aminofunktionellen Verbindungen B) umsetzt, wobei die so erhaltenen Polyurethane vor oder während Schritt B) in Wasser dispergiert werden.

5. Kosmetische Zusammensetzungen gemäß Anspruch 4, **dadurch gekennzeichnet, dass** die Polyole der Komponente A2) zahlenmittlere Molekulargewichte von 400 bis 8000 g/mol und OH-Funktionalitäten von 1,5 bis 6aufweisen.

6. Kosmetische Zusammensetzungen gemäß Anspruch 4 oder 5, **dadurch gekennzeichnet, dass** in Komponente B) eine Mischung aus Aminen ohne ionische bzw. ionogene Gruppen B1) und Aminen mit ionischen bzw. ionogenen Gruppen B2) eingesetzt werden.

7. Kosmetische Zusammensetzungen gemäß Anspruch 6, **dadurch gekennzeichnet, dass** in B1) 1,2-Ethylendiamin, Bis(4-aminocyclohexyl)methan, 1,4-Diaminobutan, Isophorondiamin, Ethanolamin, Diethanolamin, Diethylentriamin oder deren Mischungen eingesetzt werden.

8. Kosmetische Zusammensetzungen gemäß Anspruch 6 oder 7, **dadurch gekennzeichnet, dass** in B2) Amine mit Sulfonatgruppen als ionische Gruppen und zwei Aminogruppen eingesetzt werden.

9. Kosmetische Zusammensetzungen gemäß einem der Anspruch 1 bis 8, **dadurch gekennzeichnet, dass** als Vinylpyrrolidon-(Co)polymere I) Polyvinylpyrrolidon, Vinylpyrrolidon/vinylester-Copolymere, Vinylpyrrolidon/vinylacrylat-Copolymere, Terpolymere aus Vinylcaprolactam, Vinylpyrrolidon und Dimethylaminoethylmethacrylat eingesetzt werden.

10. Kosmetische Zusammensetzungen gemäß einem der Anspruch 1 bis 9, **dadurch gekennzeichnet, dass** diese bezogen auf das Gesamtgewicht der kosmetischen Zusammensetzungen 0,1 bis 20 Gew.-% an Vinylpyrrolidon-(Co)polymeren I) enthalten.

11. Kosmetische Zusammensetzungen gemäß einem der Anspruch 1 bis 10, **dadurch gekennzeichnet, dass** das relative Gewichtsverhältnis von Polyurethan II) zu Vinylpyrrolidon-(Co)polymeren I) 1 zu 10 bis 10 zu 1 beträgt.

12. Verwendung kosmetische Zusammensetzungen gemäß einem der Anspruch 1 bis 11 als Haarfestiger oder .in Haarfestigerzusammensetzungen.

13. Verfahren zum Formen von Frisuren, bei dem kosmetische Zusammensetzungen gemäß einem der Ansprüche 1 bis 11 verwendet werden.

14. Pumpspray-, Aerosolverpackungs und Schaumspender auf Pumpspray- oder Aerosolverpackungsbasis, umfassend eine oder mehrere der kosmetischen Zusammensetzungen gemäß einem der Ansprüche 1 bis 11.
